## Europäisches Patentamt

## European Patent Office

## Office européen des brevets

(19)

(11) Veröffentlichungsnummer: **0 183 021**
**B1**

(12)

# EUROPÄISCHE PATENTSCHRIFT

(45) Veröffentlichungstag der Patentschrift:
04.01.89

(51) Int. Cl.⁴: **A 61 F 5/04**

(21) Anmeldenummer: **85112847.0**

(22) Anmeldetag: **10.10.85**

(54) **Fingerschiene zur Fixierung des äussersten Fingergelenks in Streckstellung.**

(30) Priorität: **07.11.84 DE 8432615 U**

(43) Veröffentlichungstag der Anmeldung:
**04.06.86 Patentblatt 86/23**

(45) Bekanntmachung des Hinweises auf die Patenterteilung:
**04.01.89 Patentblatt 89/1**

(84) Benannte Vertragsstaaten:
**AT BE CH DE FR GB IT LI NL SE**

(56) Entgegenhaltungen:
**DE-A-3 026 839**
**US-A-2 528 456**
**US-A-2 548 378**
**US-A-2 573 715**
**US-A-3 170 460**

**D.Hohmann, R.Uhlig:"Orthopädische Technik", 7. Auflage 1982, Ferdinand Enke Verlag Stuttgart, Seite 501**

(73) Patentinhaber: **Waldemar Link GmbH & Co,**
**Barkhausenweg 10, D-2000 Hamburg 63 (DE)**

(72) Erfinder: **Link, Helmut D., Wildstieg 14, D-2000 Hamburg 65 (DE)**

(74) Vertreter: **Glawe, Delfs, Moll & Partner**
**Patentanwälte, Postfach 26 01 62 Liebherrstrasse 20, D-8000 München 26 (DE)**

LIBER, STOCKHOLM 1989

## Beschreibung

Die Erfindung bezieht sich auf eine Fingerschiene zur Fixierung des äußersten Fingergelenks in Streckstellung, bestehend aus einer das äußerste Fingergelenk und das Endglied unterseitig stützenden Schale und einer das äußerste Fingergelenk und das Mittelglied oberseitig stützenden Schale, die nahe dem äußersten Fingergelenk miteinander verbunden sind, wobei die unterseitige Schale ausgeschnitten ist bis auf einen das äußerste Fingergelenk stützenden Steg, eine den unteren Teil der Fingerkuppe aufnehmende Mulde und zwei den Steg und die Mulde miteinander verbindende Seitenwände.

Bei gebräuchlichen Fingerschienen (US-A-2 548 378; Katalog 1982 der Firma Waldemar Link GmbH & Co, Seite 39) sind die beiden Schalen im wesentlichen geschlossen ausgeführt, wenn man von einigen Bohrungen zur Luftzufuhr absieht. Der geschiente Finger wird dadurch von der Tastfunktion ausgeschlossen. Das erschwert bestimmte Tätigkeiten, für die das Tastvermögen von besonderer Wichtigkeit ist, beispielsweise das Schreiben.

Zur Vermeidung dieses Nachteils ist bei einer anderen bekannten Fingerschiene die unterseitige Schale ausgeschnitten bis auf einen das äußerste Fingergelenk stützenden Steg, eine den unteren Teil der Fingerkuppe aufnehmende Mulde und zwei den Steg und die Mulde miteinander verbindende Seitenwände (Hohmann, Uhlig: Orthopädische Technik, Stuttgart 1982, Seite 501). Dadurch wird die Palmarfläche der Fingerbeere freigelegt, so daß ihr Tastvermögen genutzt werden kann. Diese Schiene hat jedoch den Nachteil, daß das äußerste Fingerglied nur durch die die Fingerkuppe aufnehmende Mulde abgestützt ist, so daß die Gefahr entsteht, daß dieses Fingerglied bei einer versehentlichen Längsverschiebung der Schiene gegenüber dem Finger oder dann, wenn der Finger wegen eines ungewöhnlich langen Fingernagels verhältnismäßig weit hinten in der Schiene sitzt, durch die Mulde nicht hinreichend gestützt wird. Es ist deshalb eine stärkere Sicherung der Schiene am Finger mittels selbstklebender Bänder - meist auch im vorderen Endgliedbereich - notwendig, wodurch die Vorteile der Schienenausschnitte, nämlich Luftzutritt zur Haut und Nutzung der Tastmöglichkeit, eingeschränkt oder zunichte gemacht werden.

Der Erfindung liegt die Aufgabe zugrunde, diese Nachteile zu vermeiden.

Dies gelingt erfindungsgemäß dadurch, daß der Ausschnitt oval ist.

Dank der ovalen Form ist die Abstützung des Fingerendglieds nicht auf die die Fingerkuppe aufnehmende Mulde beschränkt, weil deren Kante, der ovalen Ausschnittsform folgend, zu den Seitenwänden hin nach hinten verlängert ist, so daß nicht nur die Fingerkuppe, sondern auch die seitlichen Ränder der Fingerbeere von der Schiene abgestützt werden.

Die Erfindung beruht auf der Erkenntnis, daß es für die Tastmöglichkeit der Fingerbeere nicht erforderlich ist, diese auf der gesamten Breite freiliegen zu lassen, weil auch bei seitlicher Abstützung der Fingerbeere deren weicher, fleischiger Teil gewölbt zwischen den sie abstützenden Flächen hervortreten kann. Der Ausschnitt braucht nur so groß zu sein, daß das weiche Gewebe der Fingerbeere sich in den Ausschnitt hineinlegt und hindurchwölbt. Dies ist deshalb möglich, weil das Gewebe der Fingerbeere im Vergleich mit dem der Fingerkuppe verhältnismäßig weich ist. Die Ausschnittskanten sollen innen gut ausgerundet werden, weil die Haut daran anliegt.

Auch die oberseitige Schale der Fingerschiene kann mit einem solchen Ausschnitt versehen sein, wobei dieser Ausschnitt aber weniger der Nutzung des Tastsinns als vielmehr der besseren Belüftung des umschlossenen Fingerbereichs dient.

Die Erfindung wird im folgenden näher unter Bezugnahme auf die Zeichnung erläutert, die ein vorteilhaftes Ausführungsbeispiel der Erfindung veranschaulicht. Darin zeigen:

Fig. 1    eine Seitenansicht,
Fig. 2    eine Unteransicht,
Fig. 3    eine Oberansicht und
Fig. 4    eine Vorderansicht einer Fingerschiene.

In dieser Beschreibung wird das Wort unterseitig synonym mit volar und oberseitig mit dorsal gebraucht.

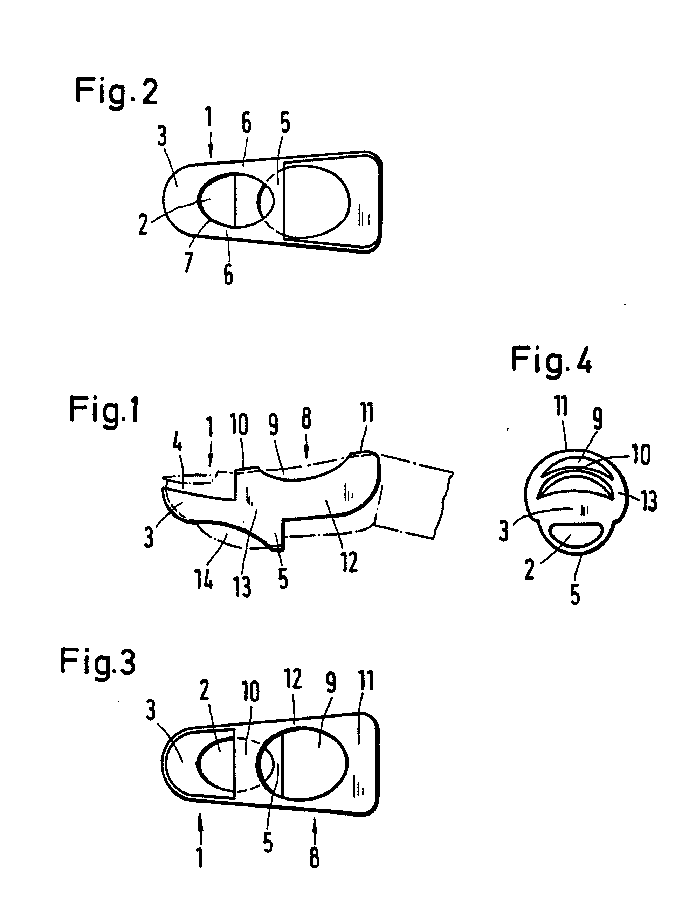

In Fig. 2 erkennt man, daß die unterseitige Schale 1 der Fingerschiene einen ovalen Ausschnitt 2 enthält, so daß außerhalb desselben am Ende eine Mulde 3 zur Stützung der Fingerkuppe 4, hinten ein Steg 5 zur Stützung des äußersten Fingergelenks und seitlich Streben 6 verbleiben, die gemeinsam eine den Ausschnitt 2 umschließende Kante 7 bilden. Die oberseitige Schale 8 der Fingerschiene kann, aber muß nicht, ebenfalls einen Ausschnitt 9 enthalten, der von den Stegen 10, 11 sowie von den Seitenteilen 12 begrenzt wird. Die beiden Schalen 1 und 8 sind im Bereich des Gelenks bei 13 in üblicher Weise einstückig miteinander verbunden. Die Lage eines Fingers ist in Fig. 1 strichpunktiert angedeutet.

Die Anlage der Mulde 3 und des Stegs 5 an der Unterseite des letzten Fingerglieds läßt das weiche Gewebe 14 auf der Unterseite desselben durch den Ausschnitt 2 kuppelartig hervordringen, so daß es trotz der beträchtlichen Dicke der Fingerschienenwand genügend vorragt, um tasten zu können.

Im Bereich des Stegs 11 wird die Schiene mittels einer Bandage am Finger befestigt.

Der Ausschnitt 2 soll so groß sein, wie die Haltefunktion der Mulde 3 und des Stegs 5 dies gestatten. Ihrer Verkleinerung sind dadurch Grenzen gesetzt, daß nur dann eine hinreichende Tastfähigkeit gegeben ist, wenn das Gewebe des Fingerendglieds noch hinreichend kuppenförmig hindurchdringen kann, derart, daß es zumindest an einer Stelle über die gerade Verbindungslinie zwischen zwei einander gegenüberliegenden äußeren Punkten der Ausschnittkanten 7 hinwegragt.

**Patentansprüche**

1. Fingerschiene zur Fixierung des äußersten Fingergelenks in Streckstellung, bestehend aus einer das äußerste Fingergelenk und das Endglied unterseitig stützenden Schale (1) und einer das äußerste Fingergelenk und das Mittelglied oberseitig stützenden Schale (8), die nahe dem äußersten Fingergelenk miteinander verbunden sind, wobei die unterseitige Schale (1) ausgeschnitten ist bis auf einen das äußerste Fingergelenk stützenden Steg (5), eine den unteren Teil der Fingerkuppe (4) aufnehmende Mulde und zwei den Steg und die Mulde miteinander verbindende Seitenwände, dadurch gekennzeichnet, daß der Ausschnitt oval ist.

2. Fingerschiene nach Anspruch 1, dadurch gekennzeichnet, daß auch die oberseitige Schale (8) bis auf zwei endständige Stege (10, 11) ausgeschnitten ist.

**Claims**

1. A finger splint for fixing the outermost finger joint in an extended position, comprising a cup (1) supporting the outermost finger joint and the end segment on the underside, and a cup (8) supporting the outermost finger joint and the medial segment on the upper side, which cups are joined together near the outermost finger joint, wherein the underside cup (1) is cut away except for a web (5) supporting the outermost finger joint, a hollow accommodating the lower part of the finger-tip (4) and two side walls joining together the web and the hollow, characterised in that the cut-away portion is oval.

2. A finger splint according to claim 1, characterised in that the upperside cup (8) is cut away except for two remaining webs (10, 11).

**Revendications**

1. Attelle pour maintenir l'articulation extrême d'un doigt en extension, constituée d'une coquille (1) soutenant par en-dessous l'articulation extrême du doigt et la phalange terminale et d'une coquille (8) maintenant par au-dessus l'articulation extrême du doigt et la phalange moyenne, ces coquilles étant reliées l'une à l'autre à proximité de l'articulation extrême du doigt, et la coquille inférieure (1) étant découpée à l'exception d'une traverse (5) soutenant l'articulation extrême du doigt, d'une cuvette (3) recevant la partie inférieure du bout du doigt (4) et de deux parois latérales reliant ensemble la traverse et la cuvette, caractérisée en ce que la découpe (2) est ovale.

2. Attelle suivant la revendication 1, caractérisée en ce que la coquille supérieure (8) est elle aussi découpée à l'exception de deux traverses (10, 11) terminales.

## Fig. 2

## Fig. 1

## Fig. 4

## Fig. 3